# EUROPEAN PATENT APPLICATION

(11) **EP 2 500 042 A1**
(43) Date of publication of application: **19.09.2012**
(21) Application number: 10829773.0
(22) Date of filing: 09.09.2010
(51) Int. Cl.: A61L 27/00, A61K 35/12, A61P 9/04, A61P 9/10

(54) **CELL SHEET FOR MYOCARDIAL REGENERATION, MANUFACTURING METHOD, AND METHOD OF USAGE THEREFOR**

(30) Priority: 13.11.2009 JP 2009276980
(71) Applicant: Sawa, Yoshiki, Hyogo, 6620099 (JP)
(72) Inventor: SHUDO, Yasuhiro, Suita-shi Osaka 565-0871 (JP); MIYAGAWA, Shigeru, Suita-shi Osaka 565-0871 (JP); SHIMIZU, Tatsuya, Tokyo 162-8666 (JP); OKANO, Teruo, Tokyo 162-8666 (JP); MATSUYAMA, Akifumi, Suita-shi Osaka 565-0871 (JP); SAITO, Atsuhiro, Suita-shi Osaka 565-0871 (JP)
(74) Representative: Wachenfeld, Joachim
(86) International application number: PCT/JP2010/065554
(87) International publication number: WO 2011/058813

(57) **Abstract**

Provided are a cell sheet and a three-dimensional structure thereof that include at least mesenchymal stem cells and myoblasts isolated from a cell culture support and that are to be applied to heart disease. Use of the cell sheet and the three-dimensional structure thereof including mesenchymal stem cells and myoblasts can notably improve cardiac functions compared with a conventional technology, i.e., use of cell sheets composed of myoblasts only or mesenchymal stem cells only. Furthermore, the cell sheet itself has high strength, and the transplantation procedure is also improved.

## Description

### TECHNICAL FIELD

The present invention relates to a three-dimensional structure that is applicable to the heart. More specifically, the invention relates to a cell sheet which comprises adult myoblasts and mensenchymal stem cells, which is applicable to the heart and which is useful in the fields of, for example, medicine, biology, drug discovery, and pharmacy, and relates to a method of producing the cell sheet and also a method of using the cell sheet.

### BACKGROUND ART

Myocardial infarction is an irreversible damage (Non-Patent Document 1). Ischemic heart disease is the cause of 50% of all death related to the cardiovascular system and is the main cause of congestive heart failure. In patients diagnosed as congestive heart failure, one-year mortality after chronic heart disease is 20% (Non-Patent Document 2). Many of currently available treatments that are used by clinicians can significantly improve the prognosis of patients suffering from acute myocardial infarction. Although angioplasty and thrombolytic agents can remove the causes of this acute myocardial infarction, the period of time from onset of occlusion to reperfusion determines the degree of irreversible myocardial damage (Non-Patent Document 3). Every drug and treatment clinically used do not show an effect of replacing a myocardial scar by functional contractile tissue. There is a demand for a novel treatment for regenerating normal myocardial cells.

Although cardiomyoplasty has been proposed as a surgical method for improving the left ventricle (LV) function of patients suffering from congestive heart failure, the effect thereof on the cardiac function is still unclear (Non-Patent Document 4). Recently, transplantation of a bioengineered heart graft using a biodegradable scaffold has been proposed as another novel strategy. This method, however, shows merely a minimum advantage in improvement of cardiac function because the graft hardly adheres to the myocardium (Non-Patent Document 5). Whether the bioengineered heart tissue can be histologically and electrically integrated in the recipient heart may be a main point in regeneration of damaged myocardium.

A main obstacle to use of exogenous tissue in transplantation of an organ (e.g., heart or blood vessel) is immune rejection. Changes occurring in an allograft and a xenograft were first reported more than 90 years ago (Non-Patent Document 6). Rejection of an artery graft pathologically causes expansion of the graft (reaching the rupture thereof) or occlusion. The former occurs through decomposition of extracellular matrix, while the latter occurs through proliferation of intravascular cells (Non-Patent Document 7). In such uses of grafts, non-biological materials are used in many cases, and harmful side effects are caused thereby.

Recently, cell transplantation has attracted attention as a therapeutic method using a biological material. Transplantation of human myoblasts into an infarcted heart, however, has the following drawbacks: 1. damage and loss of transplanted cells, 2. tissue injury of the recipient heart during transplantation, 3. efficiency in tissue supply to the recipient heart, 4. occurrence of arrhythmia, 5. difficulty in treating the entirety of the infarcted site, etc. As a result, cell transplantation cannot be said to be very successful.

Under these backgrounds, Patent Document 1 describes a novel method of culturing cells on a cell culture support, where the surface of the substrate is coated with a polymer having an upper or lower critical solution temperature of 0 to 80°C in water, the cells are cultured at a temperature not exceeding the upper critical solution temperature or not falling below the lower critical solution temperature, and the cultured cells are detached by increasing or decreasing the temperature of the substrate to exceed the upper critical solution temperature or fall below the lower critical solution temperature, without treatment with an enzyme. Patent Document 2 describes a method of culturing myocardial cells using this temperature-responsive cell culture substrate at a temperature not exceeding the upper critical solution temperature or not falling below the lower critical solution temperature and then detaching the cultured myocardial cells with low damage by increasing or decreasing the temperature of the substrate to exceed the upper critical solution temperature or fall below the lower critical solution temperature. Use of the temperature-responsive cell culture substrate has led to a variety of new developments on known culture techniques. In Patent Document 3, the technology is further developed, and it reveals that the cardiac function, which is difficult to be achieved by known technologies, can be improved over a long time using a cell sheet of myoblasts from tissues other than the heart. Furthermore, Patent Document 4 reveals that the cardiac function can be improved using a cell sheet of mesenchymal stem cells. Further therapeutic effects can be expected if the functions of these cell sheets can be improved, and there is a demand for further development.

### PRIOR ART DOCUMENTS

### PATENT DOCUMENTS

Patent Document 1: Japanese Unexamined Patent Application Publication No. Hei 02-211865
Patent Document 2: PCT Japanese Translation Patent Republication No. 2002-08387 (WO2002/008387)
Patent Document 3: PCT Japanese Translation Patent Republication No. 2005-011524 (WO2005/011524)
Patent Document 4: PCT Japanese Translation Patent Republication No. 2006-080434 (WO2006/080434)

### NON-PATENT DOCUMENTS

Non-Patent Document 1: Ho K.K., Anderson K.M., Kannel W.B., Grossman W., Levy D., Circulation, 1993, 88: 107-115
Non-Patent Document 2: American Heart Association., Dallas, Tex: American Heart Association; 2001
Non-Patent Document 3: Ryan T.J., Antman E.M., Brooks N.H., Califf R.M., Hillis L.D., Hiratzka L.F., Rapaport E., Riegel B., Russell R.O., Smith E.E. III, Weaver W.D., Gibbons R.J., Alpert J.S., Eagle K.A., Gardner T.J., Garson A. Jr, Gregoratos G., Ryan T.J., Smith S.C. Jr., J. Am. Coll. Cardiol., 1999, 34: 890-911
Non-Patent Document 4: Corin W.J., George D.T., Sink J.D., et al., J. Thorac. Cardiovasc. Surg., 1992, 104: 1662-71; Kratz J.M., Johnson W.S., Mukherjee R., et al., J. Thorac. Cardiovasc. Surg. 1994, 107: 868-78; Carpentier A., Chachques J.C., Lancet, 1985, 8840: 1267; and Hagege A.A., Desnos M., Chachques J.C., et al., Preliminary report: follow-up after dynamic cardiomyoplasty, Lancet, 1990, 335: 1122-4
Non-Patent Document 5: Leor J., Etzion S.A., Dar A., et al., Circulation, 2000, 102 [suppl. III] III-56-III-61; and Li R.K., Jia Z.Q., Weisel R.D., et al., Circulation, 1999, 100 [suppl. II]: II-63-II-69
Non-Patent Document 6: Carrel A., 1907, J. Exp. Med., 9: 226-8; Carrel A., 1912., J. Exp. Med. 9: 389-92; Calne R.Y., 1970, Transplant Proc., 2: 550; and Auchincloss, 1988, Transplantation, 46: 1
Non-Patent Document 7: Uretsky B.F., Mulari S., Reddy S., et al., 1987, Circulation, 76: 827-34

### SUMMARY OF THE INVENTION

### PROBLEMS TO BE SOLVED BY THE INVENTION

It is an object of the present invention to provide an artificial tissue or sheet that can endure transplantation surgery, can be used in actual surgery, and can be produced by culturing. It is also an object of the invention to provide a novel therapeutic method that replaces cell therapy. In particular, it is an object of the invention to produce an artificial tissue that is capable of enduring transplantation surgery, by using myoblasts and mesenchymal stem cells as materials.

### MEANS FOR SOLVING THE PROBLEMS

The present inventors have researched and developed through investigation from various angles in order to solve the above-mentioned problems and, as a result, have found that organization of a transplanted site unexpectedly progresses by using a cell sheet produced from myoblasts and mesenchymal stem cells and a three-dimensional structure thereof and that the resulting artificial tissue is easily detachable from a culture plate. The present invention has been accomplished based on these findings.

That is, the present invention provides a cell sheet comprising at least mesenchymal stem cells and myoblasts as well as a three-dimensional structure of cell sheets for the purpose of application to a heart disease. The present invention also provides a method of producing the cell sheet or the three-dimensional structure thereof on a culture substrate surface coated with a temperature-responsive polymer. The present invention is believed to be a considerably important invention that has been accomplished for the first time by using a cell sheet, namely, a cellular construct based on a novel idea that is the most unique in the world.

### ADVANTAGEOUS EFFECTS OF THE INVENTION

The use of the cell sheet containing mesenchymal stem cells and myoblasts and the three-dimensional structure of cell sheets according to the present invention notably improves the cardiac function, compared to the use of a known cell sheet composed of only myoblasts or only mesenchymal stem cells. In addition, the present invention enhances the strength of the cell sheet itself and also improves the transplantation procedure.

### BRIEF DESCRIPTION OF THE DRAWINGS

[Fig. 1] Fig. 1 is a diagram illustrating an outline of Example 1.
[Fig. 2] Fig. 2 is a diagram illustrating an outline of the procedure of Example 1.
[Fig. 3] Fig. 3 is graphs showing the amounts of mRNA expression of cell sheets cultured on the temperature-responsive culture plate in Example 1.
[Fig. 4] Fig. 4 is graphs showing the amounts of protein produced by cell sheets cultured on the temperature-responsive culture plate in Example 1.
[Fig. 5] Fig. 5 is a diagram illustrating an outline of the procedure of an animal experiment of Example 1.
[Fig. 6] Fig. 6 is a diagram showing the types of cell sheets used in Example 1.
[Fig. 7] Fig. 7 is a graph showing the results of evaluation of each cell sheet after transplantation in Example 1.
[Fig. 8] Fig. 8 is graphs showing the results of evaluation of each cell sheet after transplantation in Example 1.
[Fig. 9] Fig. 9 is graphs showing the results of evaluation of each cell sheet after transplantation in Example 1.
[Fig. 10] Fig. 10 is graphs showing the results of evaluation of each cell sheet after transplantation in Example 1.
[Fig. 11] Fig. 11 is graphs showing the results of evaluation of each cell sheet after transplantation in Example 1.
[Fig. 12] Fig. 12 is diagrams showing the results of evaluation of each cell sheet after transplantation in Example 1.
[Fig. 13] Fig. 13 is diagrams showing the results of evaluation of each cell sheet after transplantation in Example 1.
[Fig. 14] Fig. 14 is diagrams showing the results of evaluation of each cell sheet after transplantation in Example 1.
[Fig. 15] Fig. 15 is diagrams showing the results of evaluation of each cell sheet after transplantation in Example 1.
[Fig. 16] Fig. 16 is a graph showing the results of evaluation of each cell sheet after transplantation in Example 1.
[Fig. 17] Fig. 17 is a graph showing the results of evaluation of each cell sheet after transplantation in Example 1.

### DESCRIPTION OF EMBODIMENTS

The present invention relates to a cell sheet containing at least mesenchymal stem cells and myoblasts and a three-dimensional structure thereof that have been isolated from a cell culture support and are applied to heart disease. The cell sheet containing mesenchymal stem cells and myoblasts and the three-dimensional structure thereof have been revealed to be significantly useful for treatment of heart failure after myocardial infarction. The mesenchymal stem cell in the present invention is a somatic stem cell having pluripotency. The mesenchymal stem cells can be easily prepared by collecting cells from the bone marrow, adipose tissue, or other tissue of a recipient patient and culturing the cells by a known method. In the present invention, the origin of the mesenchymal stem cells is not particularly limited, but the bone marrow and adipose tissue can be easily and simply collected and are therefore preferred. The cell source is desirably somatic stem cells, which have self-propagating properties and can differentiate into myocardial cells and vascular endothelial cells, but is not limited thereto. The mesenchymal stem cells have been already shown that they can be transplanted to the heart by a known technology and can be used in actual medical treatment. Furthermore, from the viewpoints of the histocompatibility and the risk of infection in transplantation, though not limited to, somatic stem cells of a patient him/herself are particularly desirable. The mesenchymal stem cells are not limited to mesenchymal stem cells proliferated in an undifferentiated state and may be other cells differentiated from mesenchymal stem cells, such as fibroblasts, stromal cells, adipocytes, vascular endothelial cells, vascular endothelial precursor cells, smooth muscle cells, SP cells, and myocardial cells. Furthermore, the mesenchymal stem cells may contain cells that are contained in mesenchymal stem cells collected, such as stromal cells, fibroblasts, adipocytes, vascular endothelial cells, vascular endothelial precursor cell, smooth muscle cells, SP cells, and myocardial cells.

In order to achieve the present invention, myoblasts are also necessary. Any myoblast can be used, regardless of the type and the origin of tissue from which the cell is collected. For example, skeletal myoblasts of skeletal muscle tissue are abundantly present in the body and can be collected by a relatively easy procedure, and are therefore preferred. The skeletal myoblasts have been already shown that they can be transplanted to the heart by a known technology and can be used in actual medical treatment. In the present invention, the collected myoblasts may contain, in addition to mesenchymal stem cells proliferated in an undifferentiated state, other cells, such as fibroblasts, stromal cells, adipocytes, vascular endothelial cells, vascular endothelial precursor cells, and smooth muscle cells, or cells that are contained when mesenchymal stem cells are collected, such as stromal cells, fibroblasts, adipocytes, vascular endothelial cells, vascular endothelial precursor cell, smooth muscle cells, SP cells, and myocardial cells.

The present invention is based on the finding that the inherent function of myoblasts is enhanced by that both myoblasts and mesenchymal stem cells are present in a single medium. The mechanism thereof is not entirely revealed at this time, but the contact of mesenchymal stem cells with myoblasts probably enhances the function of myoblasts, or a specific protein produced by mesenchymal stem cells probably enhances the function of myoblasts. In the present invention, at least myoblasts and mesenchymal stem cells are necessary, and other cells may be present together with the myoblasts and the mesenchymal stem cells without any limitation.

An aspect to accomplish the present invention is a method of coculturing at least myoblasts and mesenchymal stem cells on a single substrate surface. The culture medium used for the coculture may be any common medium capable of culturing myoblasts and mesenchymal stem cells. Specifically, α-MEM, DMEM, F-12 medium, or a mixture thereof each supplemented with 10 to 15% autologous serum or fetal bovine serum (FBS) and an antibiotic can be used. The medium optionally contains a growth factor such as fibroblast growth factor (bFGF) or adrenomedullin. The culturing can be conducted under any appropriate conditions suitable for mammal cells, in general, at 37°C, 5% CO₂ for several days, and the culture medium is optionally replaced. Myoblasts and mesenchymal stem cells have a property that they proliferate in contact with a culture substrate and can be thereby easily separated from hematopoietic stem cells, which proliferate in a floating state. Mesenchymal stem cells can be easily confirmed by a cell surface marker such as CD29, CD44, CD71, CD90, or CD105. The cultured mesenchymal stem cells can be cryopreserved by a known method.

In the present invention, the method of coculturing at least myoblasts and mesenchymal stem cells on a single substrate surface is not particularly limited. In the simplest method, these cells in a cell suspension form are seeded on a substrate surface and are cultured. The rate of the mesenchymal stem cells contained in the suspension is preferably 5 to 95%, more preferably 10 to 80%, more preferably 15 to 60%, and most preferably 20 to 40%. If the rate of the mesenchymal stem cells is less than 5%, a sufficient effect of enhancing the functions of myoblasts in coculture cannot be expected, whereas if the rate of the mesenchymal stem cells is higher than 95%, the number of myoblasts in the resulting cell sheet is too small to yield a cell sheet exhibiting a sufficient effect. Furthermore, the coculture of these cells may be performed by culturing one of these cells first and then seeding the other thereto. Alternatively, myoblasts and mesenchymal stem cells may be cultured in a single culture medium without bringing them in contact with each other using an insert for cell culture. Thus, the culturing method is not particularly limited.

In the present invention, the thus-cultured cells including at least myoblasts and mesenchymal stem cells are collected in a sheet form and are used in regeneration of myocardial tissue. The cultured cells may be collected in a sheet form by any method, for example, by a method of culturing cells on a temperature-responsive cell culture substrate as described below, a method of using a diluted aqueous solution of a protease, a method of using an aqueous solution of a specific protease, a method of detaching cells using only an aqueous solution of EDTA, or a method of physically detaching cells using a scraper or the lke. In the present invention, a three-dimensional structure may be produced by stacking the resulting cell sheets. In such a case, the three-dimensional structure may be in any condition, for example, the three-dimensional structure may be composed of cell sheets each containing both myoblasts and mesenchymal stem cells or may be formed by stratifying a cell sheet of myoblasts and a cell sheet of mesenchymal stem cells and coculturing them. Furthermore, in the invention, the degree of contraction of cell sheets when stratified is not particularly limited, and also the other conditions for stacking have no particular limitation. The number of laminated sheets is not particularly limited, but is preferably ten or less, more preferably eight or less, and most preferably four or less. The cell density per unit area of a sheet increases by laminating cell sheets, and thereby the function as a cell sheet is also enhanced. The three-dimensional structure may be produced using a scaffold such as gel or matrigel of, for example, one or more of collagen, fibrin, and gelatin, but, though there is no restriction, these scaffolds variously behave in vivo after transplantation and are preferably not used.

The cells used in the present invention are, for example, cells directly collected from living body tissue, cells directly collected from living body tissue and differentiated in, for example, a culturing system, or a cell line, and the type of the cells is not limited. The origins of these cells are not particularly limited, and examples thereof include human, rats, mice, guinea pigs, marmosets, rabbits, dogs, cats, sheep, pigs, chimpanzees, and these animals having immunodeficiency. In the case of using the cell sheet and a three-dimensional structure thereof of the present invention, human, pig, or chimpanzee-derived cells are desirable. The medium for culturing cells in the present invention is not particularly limited and may be any culture medium that is usually used for cell culturing.

In the present invention, the number of cells seeded for culturing varies depending on the animal species of the cells used, and is usually 0.4×10⁶ to 2.5×10⁶ cells/cm², preferably 0.5×10⁶ to 2.1×10⁶ cells/cm², and more preferably 0.6×10⁶ to 1.7×10⁶ cells/cm². If the seeding density is 0.4×10⁶ cells/cm² or less, proliferation of myoblasts and mesenchymal stem cells is so low as to decrease the degree of expression of the functions of the resulting cell sheet containing myoblasts and mesenchymal stem cells. This is undesirable for performing the present invention.

The resulting cell sheet and the three-dimensional structure thereof exhibit satisfactory fibrosis inhibition, angiogenesis, or apoptosis inhibition and are therefore suitable for regeneration of myocardial tissue. In addition, the cell sheet and the three-dimensional structure thereof obtained in the present invention express a hepatocyte growth factor (HGF) and a vascular endothelial cell growth factor (VEGF) and are therefore useful for regeneration of myocardial tissue. Furthermore, it has been revealed that these effects and the yields of the HGF and the VEGF are considerably higher than those in the case of cell sheets containing myoblasts or mesenchymal stem cells separately.

In the present invention, the cell sheet and the three-dimensional structure thereof can be easily obtained by culturing the cells on a cell culture support having a surface coated with a polymer of which hydration force varies in a temperature range of 0 to 80°C. That is, the cells are cultured on the cell culture support having a surface coated with a polymer of which hydration force varies in a temperature range of 0 to 80°C, within a temperature range where the hydration force of the polymer is low. Such temperature is usually 37°C, i.e., a temperature suitable for culturing cells. The temperature-responsive polymer used in the present invention may be either a homopolymer or a copolymer. Examples of such polymers include polymers described in Patent Document 1. Specifically, the polymers are prepared by homopolymerization or copolymerization of the following monomers. Examples of the monomer that can be used include (meth)acrylamide compounds, N-alkyl or N,N-dialkyl substituted (meth)acrylamide derivatives, and vinyl ether derivatives. In the copolymer, any two or more of these monomers can be used. Furthermore, products of copolymerization with another monomer other than the above-mentioned monomers or grafting or copolymerization between polymers or mixtures of polymers and/or copolymers may be used. Crosslinking can also be employed to the extent that the inherent properties of a polymer are not impaired. In such a case, the subject to be cultured and detached is cells; hence, separation is performed in a temperature range of 5 to 50°C. Accordingly, the temperature-responsive polymer is, for example, poly-N-n-propylacrylamide (the lower critical solution temperature of a homopolymer: 21°C), poly-N-n-propylmethacrylamide (the lower critical solution temperature of a homopolymer: 27°C), poly-N-isopropylacrylamide (the lower critical solution temperature of a homopolymer: 32°C), poly-N-isopropylmethacrylamide (the lower critical solution temperature of a homopolymer: 43°C), poly-N-cyclopropylacrylamide (the lower critical solution temperature of a homopolymer: 45°C), poly-N-ethoxyethylacrylamide (the lower critical solution temperature of a homopolymer: about 35°C), poly-N-ethoxyethylmethacrylamide (the lower critical solution temperature of a homopolymer: about 45°C), poly-N-tetrahydrofurfurylacrylamide (the lower critical solution temperature of a homopolymer: about 28°C), poly-N-tetrahydrofurfurylmethacrylamide (the lower critical solution temperature of a homopolymer: about 35°C), poly-N,N-ethylmethylacrylamide (the lower critical solution temperature of a homopolymer: about 56°C), or poly-N,N-diethylacrylamide (the lower critical solution temperature of a homopolymer: 32°C). Examples of the monomer used in the present invention for copolymerization include, but not particularly limited to, acrylamide, N,N-diethylacrylamide, N,N-dimethylacrylamide, ethylene oxide, acrylic acid and salts thereof, hydroxyethyl methacrylate, hydroxyethyl acrylate, vinyl alcohol, and vinylpyrrolidone.

In the present invention, the substrate surface may be coated with a polymer by any method without particular limitation, for example, by subjecting the substrate and the above-mentioned monomer or polymer to, for example, any of irradiation with electron beams (EB), γ-rays, or ultraviolet rays, plasma treatment, corona treatment, and an organic polymerization reaction; or physical adsorption such as coating or kneading. The density of the temperature-responsive polymer coating the surface of a culture substrate can be in the range of 1.1 to 2.3 µg/cm², preferably 1.4 to 1.9 µg/cm², and more preferably 1.5 to 1.8 µg/cm². A coating density of 1.1 µg/cm² or less precludes detachment of the cells on the polymer even if a stimulus is applied, which causes a significant reduction in work efficiency and is therefore undesirable. In contrast, a coating density of 2.3 µg/cm² or more prevents the adhesion of cells to such a region and thus prevents sufficient adhesion. In such a case, if the coating layer of the temperature-responsive polymer is further coated with a cell-adhesive protein, the density of the temperature-responsive polymer coating the substrate surface may be 2.3 µg/cm² or more. The coating density of the temperature-responsive polymer in such a case is preferably 9.0 µg/cm² or less, more preferably 8.0 µg/cm² or less, and most preferably 7.0 µg/cm² or less. At a coating density of the temperature-responsive polymer of 9.0 µg/cm² or more, disadvantageously, cells hardly adhere to the surface, even if a cell-adhesive protein further coats the temperature-responsive polymer layer. The cell-adhesive protein may be of any type, and examples thereof include collagen, laminin, laminin 5, fibronectin, and matrigel. These may be used alone or as a mixture of two or more thereof. The cell-adhesive protein may be coated in accordance with a usual method, where an aqueous solution of a cell-adhesive protein is applied to a substrate surface; the aqueous solution is removed; and the surface is rinsed. The present invention provides a technology using a cell sheet itself as much as possible by utilizing a temperature-responsive culture plate. It is therefore undesirable to coat the temperature-responsive polymer layer with a significantly large density of a cell-adhesive protein. The coating density of the temperature-responsive polymer and the coating density of the cell-adhesive protein may be measured in accordance with a usual method, for example, a method of directly measuring the cell-adhering portion by FT-IR-ATR or a method of immobilizing a labeled polymer by the same method and estimating the density from the amount of the labeled polymer immobilized on the cell-adhering portion. Either method may be used.

In the present invention, the cultured cell sheet can be detached and harvested from the temperature-responsive substrate by adjusting the temperature of the culture substrate having cultured cells thereon to a temperature not lower than the upper critical solution temperature or not higher than the lower critical solution temperature of the coating polymer on the culture substrate. The harvest can be performed in the culture solution or in another isotonic solution, and these solutions can be selected depending on the purpose. In order to detach and harvest the cells more quickly and more efficiently, a method of lightly tapping or shaking the substrate and a method of stirring the culture medium with a pipette may be employed alone or in combination. Culturing conditions other than the temperature are not particularly limited and can be those in usual methods. For example, the culture medium used may be a known one, e.g., a medium containing a serum such as fetal calf serum (FCS) or a serum-free medium not containing such a serum. Furthermore, the culture medium may contain ascorbic acid or a derivative thereof for allowing the cells during cell culturing to sufficiently form an extracellular matrix.

The terms outlined above will be explained by poly(N-isopropylacrylamide) as an example of the temperature-responsive polymer. Poly(N-isopropylacrylamide) is known as a polymer having a lower critical solution temperature of 31°C and causes dehydration in water at a temperature of 31°C or higher if it is a free state to aggregate the polymer chains to cause white turbidity. On the contrary, at a temperature of lower than 31°C, the polymer chains are hydrated into a water-dissolved state. In the present invention, this polymer covers the surface of a substrate such as a petri dish and is fixed thereto. The polymer on the substrate surface is similarly dehydrated at a temperature of 31 °C or higher, and since the polymer chain covers the substrate surface and is fixed thereto, the substrate surface shows hydrophobicity. Conversely, at a temperature lower than 31°C, the polymer on the substrate surface is hydrated, while the polymer chain covers the substrate surface and is fixed thereto, thus, the substrate surface shows hydrophilicity. The hydrophobic surface is a surface suitable for adhesion and growth of cells, while the hydrophilic surface is a surface to which cells cannot adhere. Accordingly, cells during culturing or a cell sheet can be detached by merely cooling.

The substrate to be coated with a polymer may be any material that can be generally molded, such as polymer compounds other than the above-mentioned ones and ceramics, as well as compounds that are usually used in cell culturing, such as glass, modified glass, polystyrene, poly(methyl methacrylate), poly(ethylene terephthalate), and polycarbonate.

The shape of the culture substrate in the present invention is not particularly limited, and examples thereof include, but not limited to, forms such as dishes, multiplates, flasks, cell inserts, beads, and fibrous shapes; plain film forms; and porous film forms.

The cell sheet and the three-dimensional structure according to the present invention are not damaged by proteases represented by dispase and trypsin during the culture. Accordingly, the cell sheet and the three-dimensional structure thereof detached from the substrate have an adhesive protein, and a desmosome structure between cells is maintained to some extent when the cell sheet and the three-dimensional structure thereof are detached in a sheet form. This allows the cell sheet when it is transplanted to well adhere to the affected tissue, and thereby the transplantation can be efficiently performed. It is generally known that detachment with dispase, which is a protease, is possible while 10 to 40% of the desmosome structure is maintained between cells, but, for example, the basal membrane-like protein between the cells and the substrate is almost broken. As a result, the resulting cell sheet has a low strength. In contrast, the cell sheet and the three-dimensional structure thereof of the present invention maintain 60% or more of the desmosome structure and the basal membrane-like protein and, therefore, can exhibit various effects described above.

The three-dimensional structure of the present invention may be produced by any method without particular limitation. For example, the three-dimensional structure can be obtained by detaching cultured cells in the form of a sheet and stratifying the cultured cell sheets optionally using a cultured cell moving jig. In such a case, the temperature of the culture medium is not particularly limited providing that, if the polymer coating the surface of the culture substrate has an upper critical solution temperature, the temperature of the culture medium does not exceed the upper critical solution temperature while if the polymer has a lower critical solution temperature, the temperature of the culture medium does not fall below the lower critical solution temperature. Needless to say, a low temperature range in which cells cannot proliferate and a high temperature range in which cells cannot survive are inappropriate for culturing. Culturing conditions other than the temperature are not particularly limited and may be those in usual methods. For example, the culture medium to be used may be a known one, e.g., a medium containing serum such as fetal calf serum (FCS) or a serum-free medium not containing such serum. The cultured cell moving jig that can capture the detached cell sheets can be used without limitation, and examples thereof include membranes, plates and sponges, such as porous membranes, paper, and rubber. In order to facilitate the stacking procedure, a jig having a grip and provided with a membrane or plate, such as a porous membrane, paper, or rubber, or sponge may be used.

The present invention may use a carrier for facilitating adhesion of the cell sheet and the three-dimensional structure of cell sheets detached from the temperature-responsive cell culture substrate. In order to maintain the form of the cell sheet and the three-dimensional structure thereof of the present invention, for example, a polymer film, a structure molded from a polymer film, or a metal jig can be used. For example, in the case of using a polymer as a carrier material, examples of the material include polyvinylidene difluoride (PVDF), polypropylene, polyethylene, cellulose, and derivatives thereof, paper, chitin, chitosan, collagen, urethane, and gelatin. The carrier can have any shape.

The cell sheet and the three-dimensional structure thereof according to the present invention can be transplanted to a predetermined site of a living body. The transplantation site may be any portion of myocardial tissue and is not particularly limited. In another method, a particularly preferred transplantation site is the omentum, where blood vessels are abundantly present and transplantation is easy. Furthermore, the cell sheet and the three-dimensional structure thereof transplanted to the omentum may be then transplanted, in a state where blood vessels are connected, to myocardial tissue. The transplantation site may be subjected to vascular induction in advance or may not. The vascular induction may be performed by any method without particular limitation, and examples thereof include a method that involves embedding FGF, a vascular growth factor in microspheres and applying it to a living body for 8 to 10 days while changing the composition, size, and injection range of the microspheres; and a method that involves cutting polyethylene terephthalate into a bag shape having an appropriate size, putting FGF dissolved in a solution of high concentration of agarose inside the bag, and removing the bag 8 to 10 days after to produce a vascular-induced space.

Application of the cell sheet and the three-dimensional structure thereof according to the present invention to a human subject leads to long-time expression of the functions of the transplanted cell sheet and the three-dimensional structure thereof in the human body. The expression level of the function can be controlled by the sizes and/or shapes of a detached cell sheet and a detached three-dimensional structure thereof. Such a cell sheet and a three-dimensional structure thereof can be used for, though not particularly limited to, treating disorders accompanied by diseases or damages selected from the group consisting of heart failure, ischemic heart disease, myocardial infarction, cardiomyopathy, myocarditis, hypertrophic cardiomyopathy, dilated phase of hypertrophic cardiomyopathy, and dilated cardiomyopathy.

If the cell sheet and the three-dimensional structure thereof according to the present invention are transplanted to an animal, the animal can be used for drug evaluation. The expression level of the functions can be controlled by the sizes and/or shapes of a detached cell sheet and a detached three-dimensional structure thereof. Examples of the animal to be used include, but not particularly limited to, rats, mice, guinea pigs, marmosets, rabbits, dogs, pigs, chimpanzees, and these animals having immunodeficiency. Such transplantation animals are used for, for example, a cardiac function evaluation system for judging an effect of a test agent administered to an animal on the cardiac function, but the use is not particularly limited.

### EXAMPLES

The present invention will now be described in more detail based on examples, but should not be limited thereto.

In the examples, animals were handled by complying with standards specified by Osaka University, and experiments were performed according to animal care moral.

### Example 1

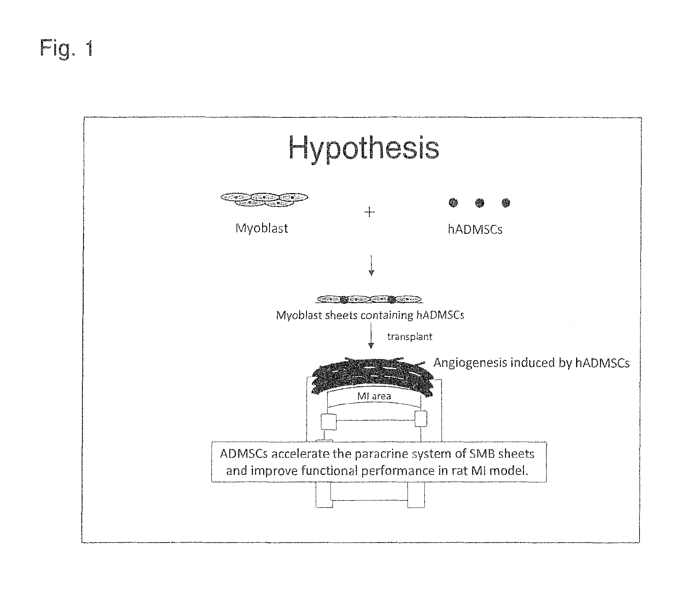

Human adipocyte-derived mesenchymal stem cells (hereinafter referred to as ADMSCs), which have been reported to have a strong angiogenesis effect, were used under the expectation of enhancement of the cytokine-producing ability of a conventional myoblast sheet. Accordingly, it was investigated whether ADMSCs enhance the paracrine effect of myoblasts to improve the cardiac function of a myocardial infarction rat model (Fig. 1).

A 35-mm polystyrene cell culture plate (manufactured by Corning Inc.) was coated with poly-N-isopropylacrylamide, a temperature-responsive polymer, and was used as a cell culture substrate. The temperature-responsive polymer was performed applied in accordance with the method described in Patent Document 1 to give a temperature-responsive cell culture substrate coated with 1.9 µg/cm² of the temperature-responsive polymer. On this surface, a 4:1 mixture of the above-described myoblasts (hereinafter referred to as SMBs) and ADMSCs was seeded at 5.7×10⁵ cells/cm² and was cultured as it was. The substrate surface coated with poly-N-isopropylacrylamide exhibits hydrophobicity at 32°C or more and hydrophilicity at less than 31°C. Consequently, adherent culture was performed under culturing conditions of a temperature of 37°C, and the cells were detached in a sheet form at a lower temperature than 31°C without trypsin treatment.

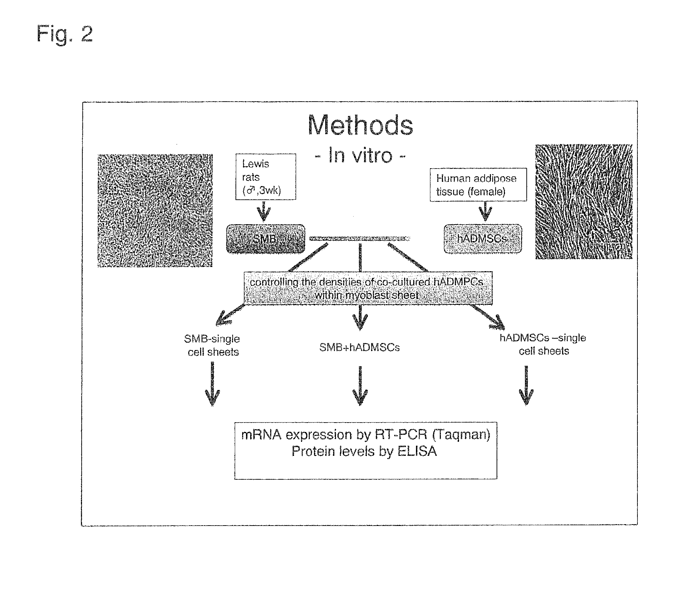

The experiment was performed in accordance with the procedure shown in Fig. 2. SMBs were isolated from the crural muscle of three-week old Lewis rats (male) and were cultured. Human ADMSCs were isolated from adult female subcutaneous adipose and were cultured. Skeletal muscle myoblasts were collected by washing both anterior tibials of a three-week old male rat with a Hank's balanced salt solution containing penicillin, streptomycin, and amphotericin B to remove fibrous tissue, tendinous tissue, and adipose tissue as much as possible, performing enzymatic digestion with collagenase type II and trypsin-EDTA for 30 min, and collecting cells from the enzymatic digestion solution by centrifugation. The myoblasts were cultured in a Dulbecco's modified Eagle medium containing 20% fetal bovine serum at 37°C, 5% CO₂. Human adipose-derived mesenchymal stem cells were collected by cutting subcutaneous adipose into small pieces, enzymatically digesting the adipose with collagenase, and collecting cells from the enzymatic digestion solution by centrifugation. The mesenchymal stem cells were cultured in a Dulbecco's modified Eagle medium at 37°C, 5% CO₂. After the culturing, SMBs and hADMSCs were mixed at a ratio of 4:1. The cell mixture was seeded on a temperature-responsive culture plate to form a cell sheet. HGF in the cell sheet, mRNA expression of VEGF, and cytokine produced in the supernatant of the sheet were quantitatively determined. A myocardial infarction model was formed by ligating the left anterior descending coronary artery of each athymic rat (female, 8-week old, 120 to 130 g). After two weeks, the rats were separated into four groups: (1) group S: SMB-single cell sheet transplantation (3 sheets) (n = 5), (2) group M: SMB/hADMSC mixture sheet transplantation (3 sheets) (SMB:hADMSCs = 4:1) (n = 6), (3) group A: hADMSC-single cell sheet transplantation (3 sheets) (n = 6), and (4) group C: Sham operation (n = 4). After 2, 4, and 6 weeks from the transplantation, echocardiography was performed.

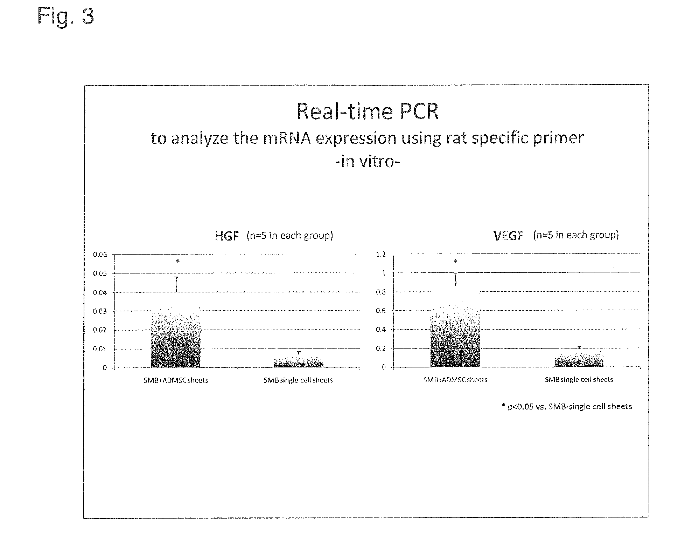

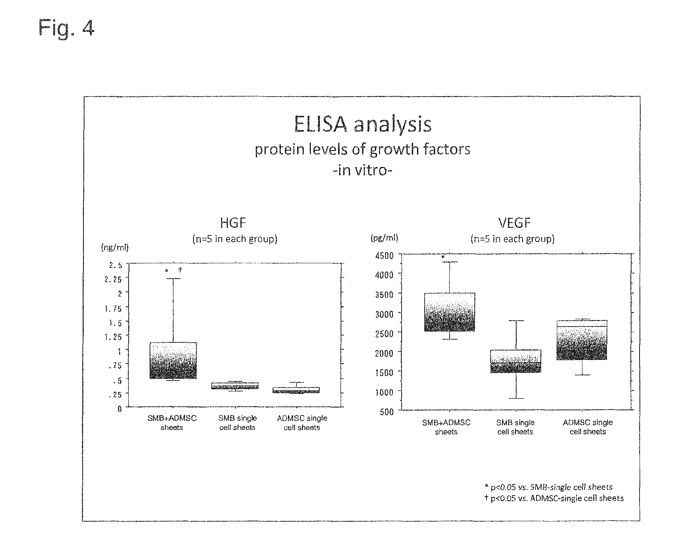

The results of RT-PCR of the culture media using rat specific primers show that expression levels of HGF and VEGF of the SMB/hADMSC mixture sheet, namely, the cell sheet of the present invention are significantly higher than those of the SMB-single cell sheet (Fig. 3). The results of ELISA of the supernatants of the culture media show that HGF production of the SMB/ADMSC mixture sheet is significantly higher than those of the other cell sheets and that VEGF production of the SMB/ADMSC mixture sheet is significantly higher than that of the SMB-single cell sheets (Fig. 4).

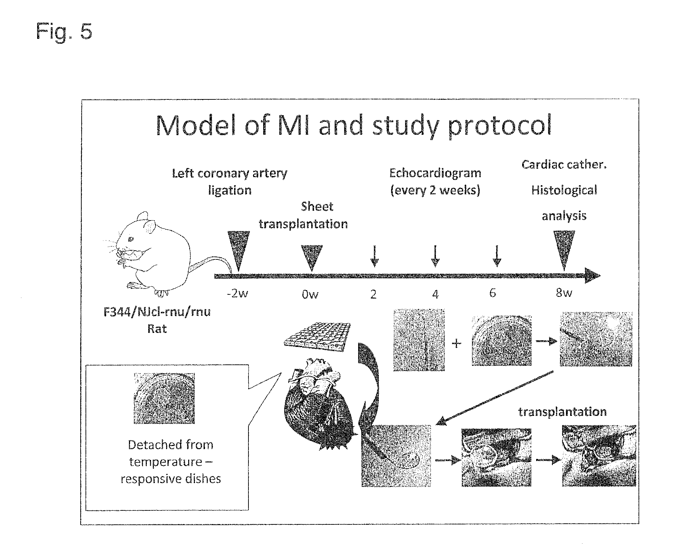

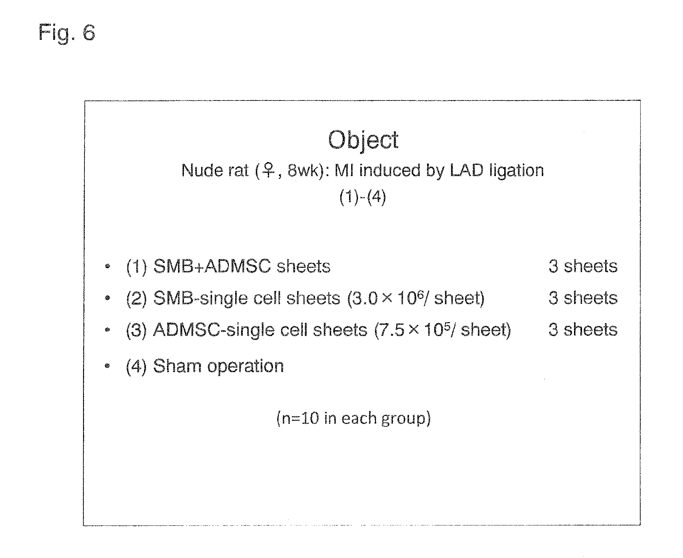

In accordance with a usual method, a myocardial infarction model was formed by ligating the left anterior descending coronary artery of each athymic rat (female, 8-week old). After 2 weeks, a cell sheet was transplanted to the affected part. At 2, 4, 6, and 8 weeks after the transplantation, cardiac function was evaluated by echocardiography. After 8 weeks, a cardiac catheter test was performed, and the rats were sacrificed for a histopathological investigation (Fig. 5). As shown in Fig. 6, the investigation was performed in four groups: an SMB/ADMSC mixture sheet group, an SMB-single cell sheet group, an ADMSC-single cell sheet group, and a Sham group.

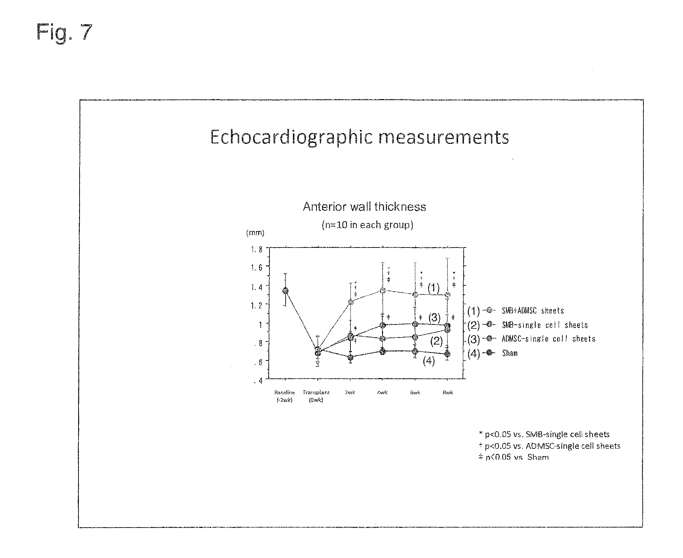

Fig. 7 shows the results of an echocardiographic change with time in thickness of left ventricle anterior wall. The thickness in the SMB/ADMSC mixture sheet group was significantly larger than those in the SMB-single cell sheet and ADMSC-single cell sheet groups at each time point of 2, 4, 6, and 8 weeks after the transplantation. In addition, the thickness in the SMB-single cell sheet group was significantly large compared with that in the Sham group at each time point of 2, 4, 6, and 8 weeks after the transplantation. In the ADMSC-single cell sheet group, the thickness at the time point of 2 weeks after the transplantation was significantly larger than that in the Sham group, but after that no significant difference was obtained.

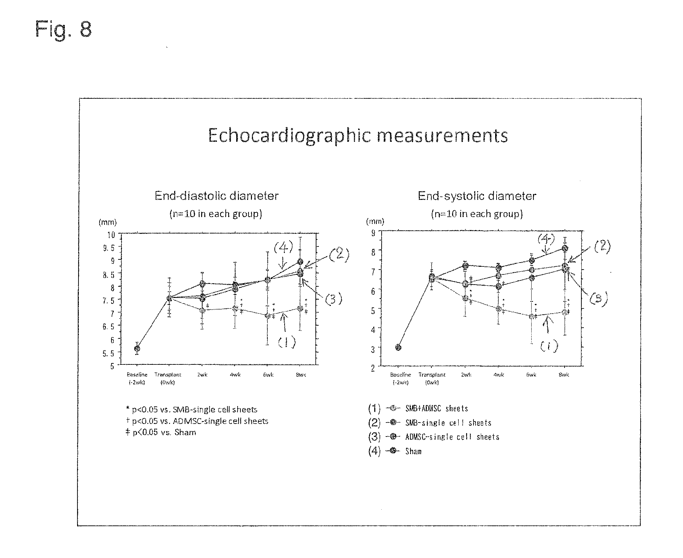

Fig. 8 shows changes with time in left ventricle end-diastolic diameter and end-systolic diameter. The left ventricle end-diastolic diameter in the Sham group significantly increased at each time point of 2, 4, 6, and 8 weeks after the transplantation compared with the diameter at the time of sheet transplantation. The SMB group exhibited a significant increase in the diameter at each time point of 6 and 8 weeks after the transplantation. In contrast, the ADMSC-single cell sheet and SMB/ADMSC mixture sheet groups exhibited no significant difference. Similarly, the left ventricle end-systolic diameter in the Sham group significantly increased with time after transplantation. Also in the SMB group, the end-systolic diameter significantly increased at the time point of 8 weeks after the transplantation. In the ADMSC group, no significant difference was observed. In contrast, in the SMB/ADMSC mixture sheet group, significant decrease was observed at 4, 6, and 8 weeks after the transplantation. That is, the left ventricle end-diastolic diameter in the SMB/ADMSC mixture sheet transplantation group was significantly smaller at each time point of 4, 6, and 8 weeks after the transplantation than those in other groups. The SMB-single cell sheet group and the ADMSC-single cell sheet group did not exhibit significant differences from the Sham group. The left ventricle end-systolic diameter in the SMB/ADMSC mixture sheet transplantation group was significantly smaller at each time point of 2, 4, 6, and 8 weeks after the transplantation than those in other groups. In the SMB sheet transplantation group, the diameter at each time point of 2 and 4 weeks after the transplantation was significant smaller than that in the Sham group. In the ADMSC-single cell sheet transplantation group, the diameter at the time point of 2 weeks after the transplantation was significant smaller than that in the Sham group, but after that, no significant difference was observed.

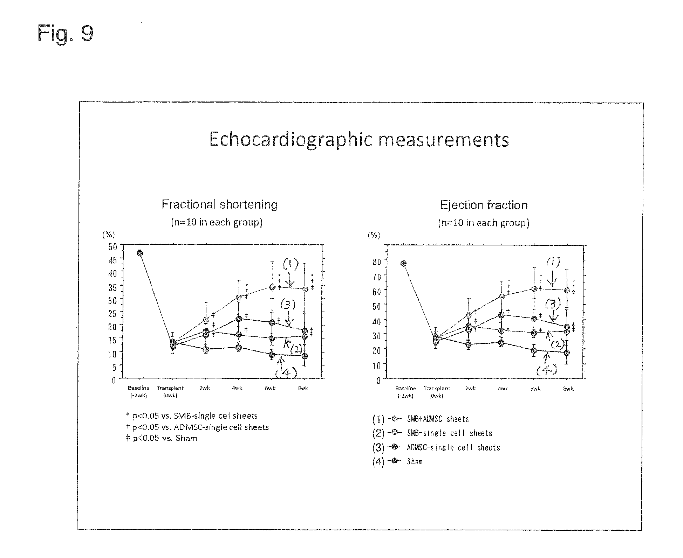

Fig. 9 shows changes over time in fractional shortening (FS) and ejection fraction (EF). The FS in the Sham group significantly decreased at each time point of 6 and 8 weeks after the sheet transplantation compared with that at the time of the transplantation. In the SMB group, the FS is significantly improved at each time point of 2 and 8 weeks after the transplantation compared with that at the time of sheet transplantation. In the ADMSC-single cell sheet group and the mixture sheet group, the FS is significantly improved at any time point of 2, 4, 6, and 8 weeks after the transplantation compared with that at the time of the transplantation. It was found that changes in the EF also showed the similar behavior. That is, the results show that the FS of the SMB/ADMSC mixture sheet transplantation group was significantly higher than that in the Sham group at the time point of 2 weeks after the transplantation and also significantly higher than those in the other three groups at each time point of 4, 6, and 8 weeks after the transplantation. Also in the ADMSC-single cell sheet transplantation group, the FS was significantly higher than that in the Sham group at each time point of 2, 4, 6, and 8 weeks after the transplantation. In the SMB-single cell sheet transplantation group, the FS was significantly higher than that in the Sham group at each time point of 2, 4, and 8 weeks after the transplantation. The EF also showed the similar behaviors.

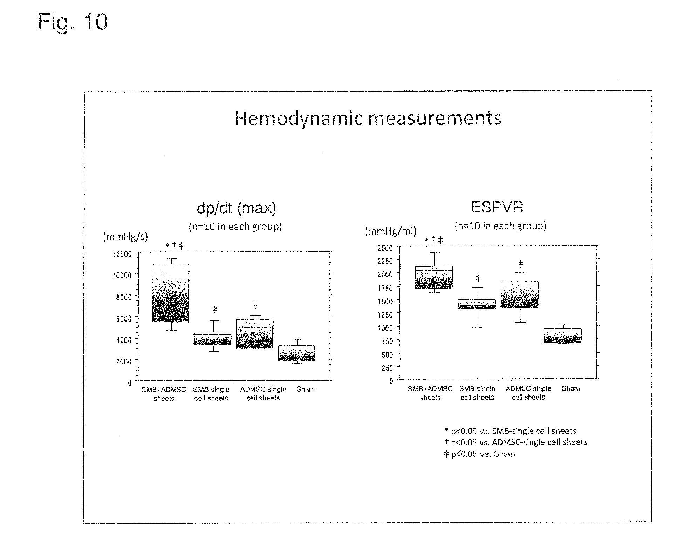

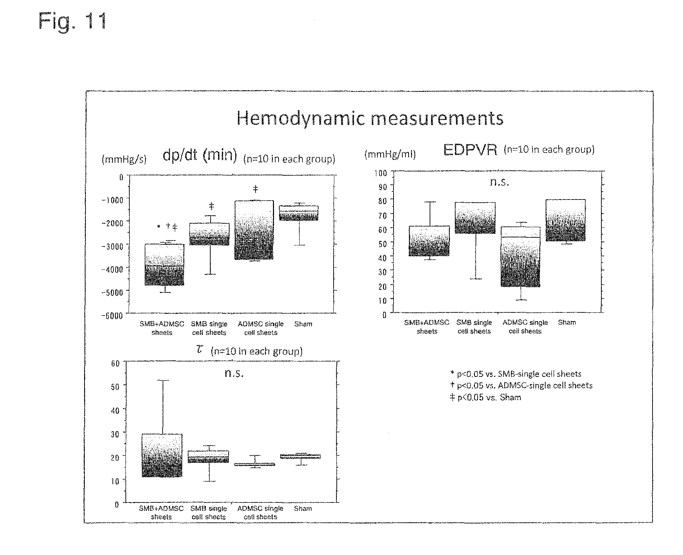

Fig. 10 shows parameters calculated in a cardiac catheter test. Systolic performance indices, dp/dt max and ESPVR, were both significantly higher in the SMB/ADMSC mixture sheet group than those in the other groups and were significantly higher in the SMB-single cell sheet group and the ADMSC-single cell sheet group than those in the Sham group. A diastolic performance index, dp/dt min, was significantly higher in the SMB/ADMSC mixture sheet group than those in the other groups and was significantly higher in the SMB-single cell sheet group and the ADMSC-single cell sheet group than that in the Sham group. No significant difference was observed in EDPVR, τ (Fig. 11).

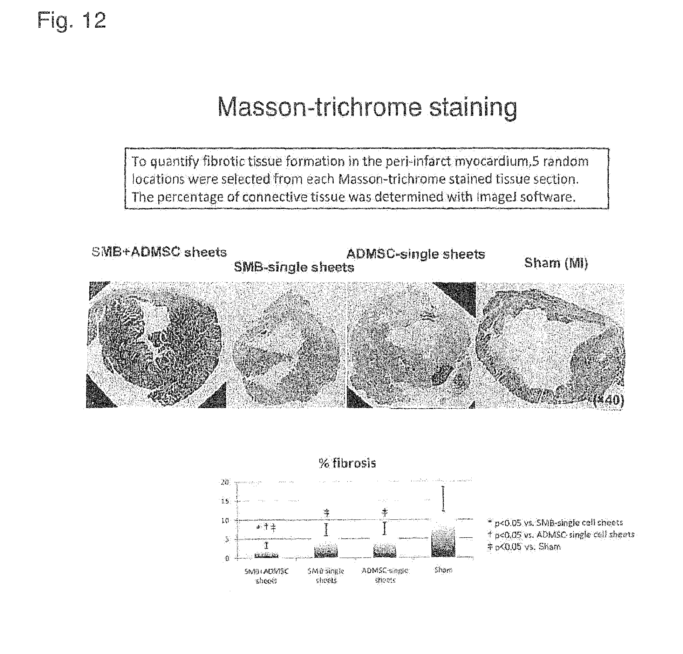

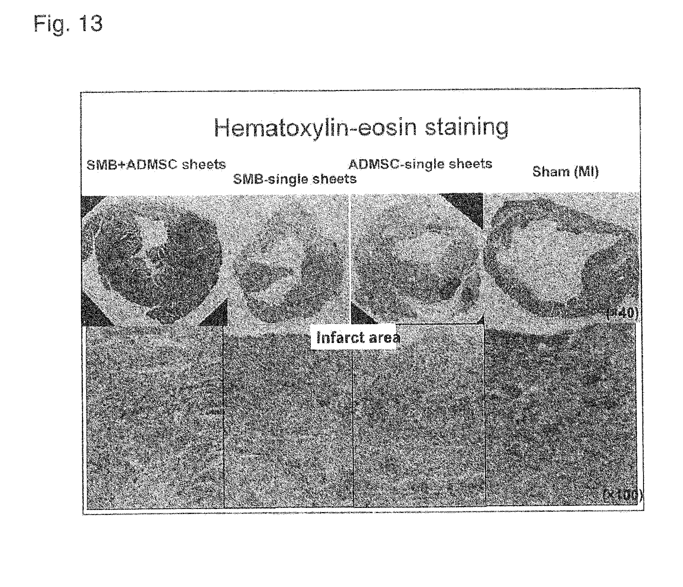

Fibrosis rates of peri-infarct areas were quantitatively determined by Masson-trichrome staining (Fig. 12). The fibrosis rate was significantly lower in the SMB/ADMSC mixture sheet group than those in the other groups and also significantly lower in the SMB-single cell sheet group and the ADMSC-single cell sheet group than that in the Sham group. The results of hematoxylin-eosin staining also show that the cardiac wall in the SMB/ADMSC mixture sheet is thicker than those in other groups (Fig. 13).

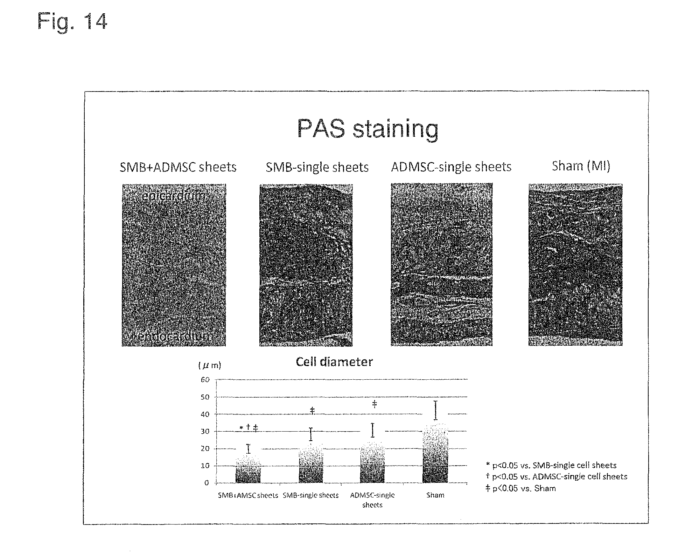

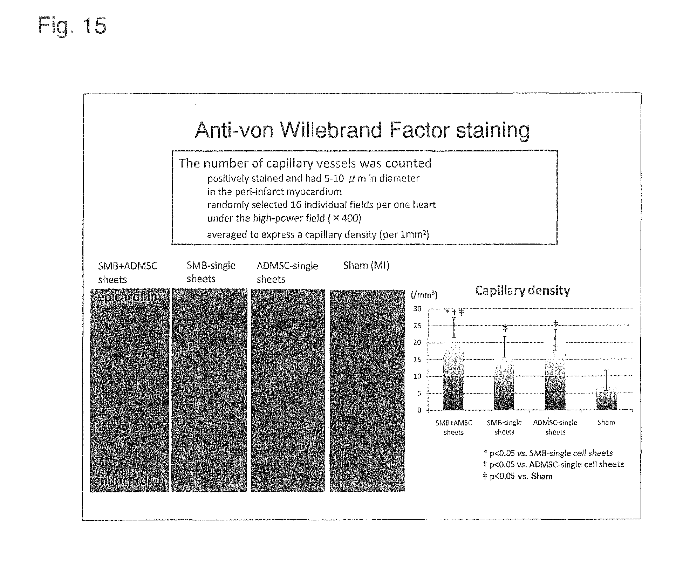

The cell diameter of each sheet transplanted site was measured using the figures of PAS staining (Fig. 14). The cell diameter was significantly smaller in the SMB/ADMSC mixture sheet group than those in the other groups and also significantly smaller in the SMB-single cell sheet group and the ADMSC-single cell sheet group than that in the Sham group. The capillary density of each peri-infarct area was quantitatively measured by Factor 8 staining (Fig. 15). The capillary density was significantly higher in the SMB/ADMSC mixture sheet group than those in the other groups and also significantly higher in the SMB-single sheet group and the ADMSC-single sheet group than that in the Sham group.

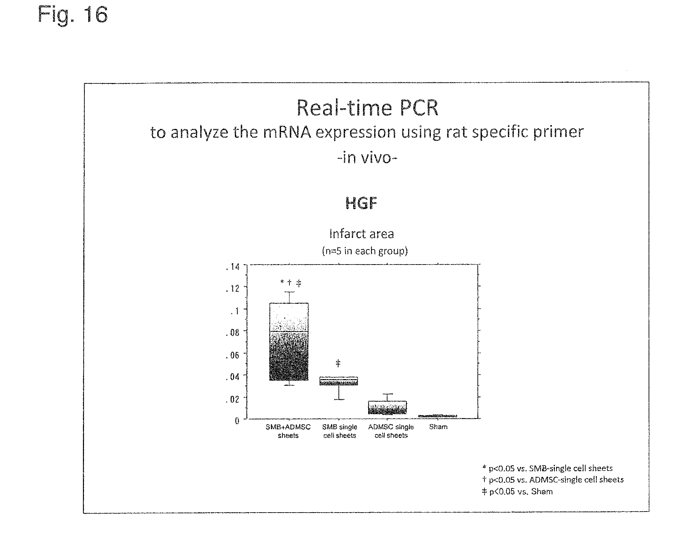

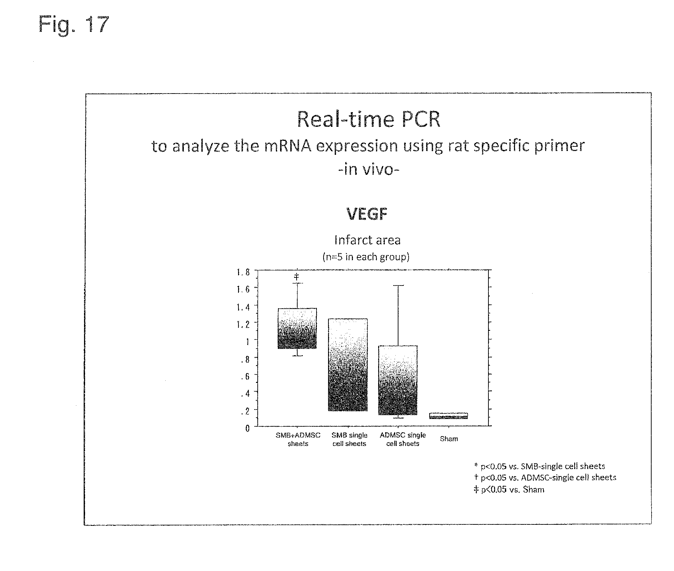

Fig. 16 shows the RT-PCR results of cut-out myocardium at 8 weeks after the transplantation. The HGF expression in vivo in the infarct area was significantly higher in the SMB/ADMSC mixture sheet group than those in the other groups and also significantly higher in the SMB-single cell sheet group than that in the Sham group. The HGF expression in vivo in a remote area was significantly higher in the SMB/ADMSC mixture sheet group than that in the Sham group. The VEGF expression in vivo in the infarct area (and also in a remote area) was significantly higher in the SMB/ADMSC mixture sheet group than that in the Sham group (Fig. 17).

These results reveal that a mixture of myoblasts and adipocyte-derived mesenchymal stem cells enhances cytokine production, thereby improving the cardiac function of a rat myocardial infarction model. The mechanism thereof is unclear at this time, but it is understood that the adipocyte-derived mesenchymal stem cells themselves directly enhance the cytokine-producing ability of a conventional myoblast sheet, or that a specific protein produced by ADMSCs enhances the cytokine-producing ability.

### INDUSTRIAL APPLICABILITY

The use of the cell sheet and the three-dimensional structure thereof comprising mesenchymal stem cells and myoblasts shown in the present invention notably enhances cardiac functions, compared with the use of a known technology, namely, a cell sheet composed of either myoblasts only or mesenchymal stem cells only. Further therapeutic effects can be expected through use of such a cell sheet and three-dimensional structure thereof.

## Claims

1. A cell sheet and a three-dimensional structure thereof to be applied to heart disease, comprising at least mesenchymal stem cells and myoblasts isolated from a cell culture support.

2. The cell sheet and the three-dimensional structure thereof according to Claim 1, wherein the cell sheet comprises a mixture of mesenchymal stem cells and myoblasts.

3. The cell sheet and the three-dimensional structure thereof according to Claim 2, wherein the mesenchymal stem cells are contained in an amount of 5 to 95%.

4. The cell sheet and the three-dimensional structure thereof according to any one of Claims 1 to 3, wherein the three-dimensional structure is a stack of a plurality of layers of the cell sheet.

5. The cell sheet and the three-dimensional structure thereof according to Claim 4, wherein the three-dimensional structure is a stack of a cell sheet comprising myoblasts and a cell sheet comprising mesenchymal stem cells.

6. The cell sheet and the three-dimensional structure thereof according to any one of Claims 1 to 5, having at least one function selected from the group consisting of fibrosis inhibition, angiogenesis, and apoptosis inhibition.

7. The cell sheet and the three-dimensional structure thereof according to any one of Claims 1 to 6, wherein the mesenchymal stem cells are derived from adipose tissue.

8. The cell sheet and the three-dimensional structure thereof according to any one of Claims 1 to 7, wherein the myoblasts are derived from skeletal muscle tissue.

9. The cell sheet and the three-dimensional structure thereof according to any one of Claims 1 to 8, wherein no scaffold is used.

10. The cell sheet and the three-dimensional structure thereof according to any one of Claims 1 to 9, wherein the cells are derived from a subject to which the cell sheet and the three-dimensional structure thereof are applied.

11. A drug to be applied to heart disease, the drug comprising the cell sheet and the three-dimensional structure thereof according to any one of Claims 1 to 10.

12. The drug according to Claim 11, the drug being used in combination with cytokine or a growth factor.

13. The drug according to Claim 11 or 12, wherein the heart disease isselected from the group consisting of heart failure, ischemic heart disease, myocardial infarction, cardiomyopathy, myocarditis, hypertrophic cardiomyopathy, dilated phase of hypertrophic cardiomyopathy, and dilated cardiomyopathy.

14. A method of producing a cell sheet and a three-dimensional structure thereof comprising at least mesenchymal stem cells and myoblasts for application to heart disease without using any scaffold, the method comprising the steps of:
a) culturing cells on a cell culture support coated with a temperature-responsive polymer having an upper critical solution temperature or a lower critical solution temperature of 0 to 80°C in water;
b) adjusting the temperature of the culture solution to a temperature not lower than the upper critical solution temperature or not higher than the lower critical solution temperature; and
c) detaching the cultured cells from the cell culture support as a cell sheet and a three-dimensional structure thereof.

15. The method according to Claim 14, wherein ascorbic acid or a derivative thereof is added to the culture medium before the detachment.

16. The method according to Claim 14 or 15, wherein no protease treatment is performed during or before the detachment.

17. The method according to any one of Claims 14 to 16, wherein the temperature-responsive polymer is poly(N-isopropylacrylamide).
